# EUROPEAN PATENT APPLICATION

(11) **EP 4 491 705 A1**
(43) Date of publication of application: **15.01.2025**
(21) Application number: 23787596.8
(22) Date of filing: 07.04.2023
(51) Int. Cl.: C12N 1/06, C12N 1/00, C12P 17/18, C12P 17/12, A23K 10/16, A23L 33/145, A23L 31/15

(54) **YEAST EXTRACT WITH HIGH BASE AND BASE DERIVATIVE CONTENTS AND METHOD FOR PREPARING SAME**

(30) Priority: 11.04.2022 CN 202210376038
(71) Applicant: Angel Yeast Co., Ltd, Yichang, Hubei 443003 (CN)
(72) Inventor: QIN, Xianwu, Yichang, Hubei 443003 (CN); ZHANG, Yan, Yichang, Hubei 443003 (CN); WU, Yexu, Yichang, Hubei 443003 (CN); LEI, Senlin, Yichang, Hubei 443003 (CN); DING, Yongzhi, Yichang, Hubei 443003 (CN); CHEN, Wenlong, Yichang, Hubei 443003 (CN)
(74) Representative: karo IP
(86) International application number: PCT/CN2023/086885
(87) International publication number: WO 2023/197952

(57) **Abstract**

Provided are a yeast extract with high base and base derivative contents and a method for preparing same. The yeast extract with high base and base derivative contents provided herein comprises 20000-40000 mg/kg of bases and base derivatives. The yeast extract provided herein is rich in bases and base derivatives that can be more easily absorbed by microorganisms than nucleotides, thus having a high effect on promoting microbial growth.

## Description

### Technical Field

The present disclosure belongs to the technical field of biological products, and particularly relates to a yeast extract with high base and base derivative contents and a method for preparing same.

### Background Art

Yeast extract is a powder, paste, or liquid product obtained by using fresh yeast as raw material, inducing various enzymes in yeast through modern biotechnological means or changing the medium environment, using certain exogenous enzymes to promote the degradation of intracellular proteins and nucleic acids under appropriate conditions, and then undergoing some refining processes. It is rich in proteins, amino acids, peptides, nucleotides, B vitamins, and trace elements, with the main role of supplementing nitrogen sources and providing various vitamins, amino acids, and growth factors for microbial growth. The yeast extract has been widely used in fermentation medium industries and is a culture medium material with a strong growth-promoting ability.

Unlike nucleotides, bases (nucleobases) and base derivatives (nucleobase derivatives) are products of further degradation of nucleotides and are constitutional units of yeast nucleic acids, which can be obtained by degradation of yeast nucleic acids during yeast autolysis. Base derivatives are products of further degradation of bases. Enzymes that degrade yeast nucleic acids can be activated during yeast autolysis to degrade the yeast nucleic acids into bases and base derivatives.

### Summary of the Invention

A yeast extract with high base and base derivative contents can provide special growth factors for microorganisms and can significantly promote microbial growth. Moreover, it can also promote microbial metabolism to produce some pharmaceutical intermediates, such as adenosine, inosine, etc.

In the existing yeast extracts, yeast nucleic acids are only degraded to the level of nucleotides but not degraded into bases and base derivatives. Therefore, the existing yeast extracts have a very low content of bases and base derivatives, which cannot meet the requirements of efficient production in microbial fermentation enterprises.

In order to solve the technical problem of the low content of bases and base derivatives in yeast extracts in the prior art, the present disclosure provides a yeast extract with high base and base derivative contents.

The content of bases and base derivatives in the yeast extract provided herein is greater than 20000 mg/kg.

In a first aspect, the present disclosure provides a yeast extract with high base and base derivative contents, the yeast extract comprising 20000-40000 mg of bases and base derivatives per kg of yeast extract.

Preferably, the yeast extract comprises 22000-39000 mg of bases and base derivatives per kg of yeast extract;
preferably, the bases and base derivatives are one or two or more selected from the group consisting of adenine, guanine, uracil, cytosine, xanthine, and hypoxanthine;
preferably, the yeast extract comprises 2000-5000 mg of adenine per kg of yeast extract;
and/or, preferably, the yeast extract comprises 3000-6000 mg of guanine per kg of yeast extract;
and/or, preferably, the yeast extract comprises 4000-7000 mg of uracil per kg of yeast extract;
and/or, preferably, the yeast extract comprises 1500-5000 mg of cytosine per kg of yeast extract;
and/or, preferably, the yeast extract comprises 8000-11000 mg of xanthine per kg of yeast extract;
and/or, preferably, the yeast extract comprises 2000-5000 mg of hypoxanthine per kg of yeast extract.

Preferably, the yeast extract comprises a total nitrogen content of 10-12% by weight; preferably, the yeast extract comprises the total nitrogen content of 10.5-11.8% by weight;
preferably, the yeast extract comprises a nitrogen content from amino acids of 3.5-5.5% by weight; preferably, the yeast extract comprises the nitrogen content from amino acids of 4-5% by weight. In a second aspect, the present disclosure provides a method for preparing the yeast extract, comprising the following steps:
heating to 40-60°C, adjusting the pH to 5.0-6.0, adding the content of an enzyme of 0.1-1% by weight based on the dry weight of the yeast milk, adding the content of a precursor of 1-5% by weight based on the dry weight of the yeast milk, and autolyzing for 15-24 h.

The precursor described herein is converted into bases and base derivatives during autolysis. Preferably, the enzyme preparation is one or two or more selected from the group consisting of nuclease, adenine deaminase, and guanine deaminase,
preferably, the enzyme preparation is a combination of nuclease, adenine deaminase, and guanine deaminase;
preferably, the mass ratio of the nuclease, adenine deaminase, and guanine deaminase is 2.5-5:1:1. Preferably, the precursor is one or two selected from the group consisting of yeast RNA or disodium ribonucleotide.

Preferably, the method further comprises: inactivating the enzyme after autolysis, solid-liquid separation, and then concentrating the liquid to obtain the yeast extract;
preferably, the enzyme is inactivated at 65-75°C;
preferably, the concentration is performed by evaporation under reduced pressure.

Preferably, the yeast is selected from one or two or more selected from the group consisting of *Saccharomyces cerevisiae, Wickerhamomyces anomalus,* and *Cyberlindnera fabianii.*

Preferably, the method for preparing the yeast milk comprises the following steps:
inoculating a yeast strain into a culture medium, culturing at pH 4.0-6.0 and a temperature of 30-33°C for 12-24 h, and collecting yeast cells to obtain the yeast milk.

Preferably, in the yeast milk, the content of the yeast cells is 10-15 wt% based on dry matter content, and the rest is water.

Preferably, the inoculation amount is 1-5 wt%,
preferably, the culture medium is obtained by adding 3-10 g of carbon source, 0.5-1 g of yeast extract, 2-5 g of ammonium sulfate, 1-2 g of magnesium sulfate, 0.5-1 g of potassium dihydrogen phosphate, and 0.1-0.5 g of zinc sulfate to every 100 ml of water,
preferably, the carbon source is one or two or more selected from the group consisting of cane molasses, beet molasses, or hydrolyzed sugars.

In a third aspect, the present disclosure further provides a yeast extract prepared by the above method.

In a fourth aspect, the present disclosure further provides the use of the yeast extract or a yeast extract prepared by the above method in foods, feeds, and culture media.

The yeast extract is an organic nitrogen source with very comprehensive nutrition, which can provide a nitrogen source for microbial growth and growth factors such as trace elements, vitamins, etc. The traditional yeast extract is rich in nucleotides. In contrast, the yeast extract provided herein is rich in bases and base derivatives that can be more easily absorbed by microorganisms than nucleotides, thus having a high effect on promoting microbial growth and good quality compared to the traditional yeast extract. The method provided herein can significantly increase the content of bases and base derivatives in a yeast extract.

### Detailed Description of the Invention

It is an object of the present disclosure to provide a yeast extract with high base and base derivative contents. The existing yeast extracts have a low content of bases and base derivatives, and have insufficient ability to promote microbial growth and microbial metabolic production of products, which cannot meet the requirements of efficient production in microbial fermentation enterprises. The yeast extract provided herein is rich in bases and base derivatives and has a high effect on promoting microbial growth and metabolism. According to the method provided herein, an enzyme preparation and a substrate are added to yeast milk, and the yeast autolysis and the degradation of yeast nucleic acids into bases and base derivatives are promoted by controlling temperature, pH, and other conditions to obtain a yeast extract with high base and base derivative contents.

The content of bases and base derivatives in the yeast extract provided herein is greater than 20000 mg/kg. The bases are one or two or more selected from the group consisting of adenine, guanine, uracil, and cytosine; the base derivatives are one or two of hypoxanthine and xanthine.

A method for preparing the yeast extract provided herein comprises: autolyzing and enzymolyzing yeast milk, adding an enzyme preparation to the yeast milk, and autolyzing at 40-60°C and pH 5.0-6.0 for 15-24 h. In a preferred embodiment of the present disclosure, the method further comprises separating and purifying the product obtained by autolysis, preferably separating the yeast autolysis solution through solid-liquid separation, concentrating the liquid to obtain the yeast extract, preferably evaporating under reduced pressure to obtain a paste yeast extract, or spray-drying to obtain a powder yeast extract. In a specific embodiment of the present disclosure, the evaporation under reduced pressure is performed at 92-95 kpa and 80°C.

In a specific embodiment of the present disclosure, the method for preparing the yeast extract comprises the following steps:
1) Obtaining yeast milk: fermentation condition: pH 4.0-6.0, culture temperature: 30-33°C, culture time: 12-24 h. The fermentation medium is formulated as follows: 3-10% of carbon source, 0.5-1% of yeast extract, 2-5% of ammonium sulfate, 1-2% of magnesium sulfate, 0.5-1% of potassium dihydrogen phosphate, and 0.1-0.5% of zinc sulfate (all above in mass to volume ratio w/v). The carbon source is one or two or more selected from the group consisting of cane molasses, beet molasses, or hydrolyzed sugars. At the end of fermentation, the fermentation broth is centrifuged at 5000-7000 rpm, and the supernatant is discarded. Purified water is added to the heavy phase and then centrifuged again to obtain a washed yeast. The yeast is diluted with purified water to produce yeast milk with a dry matter content of 10-15%.
2) Yeast autolysis: heating the yeast milk to 40-60°C and maintaining at this temperature, adjusting the pH to 5.0-6.0, adding 0.1-1% of an enzyme preparation based on the dry weight of the yeast milk, adding 1-5% of a substrate based on the dry weight of the yeast milk, and autolyzing for 15-24 h. The enzyme preparation is nuclease, adenine deaminase, and guanine deaminase. The substrate is yeast RNA or disodium ribonucleotide.
3) Separating and purifying: inactivating the enzyme in the autolytic solution obtained after the above yeast autolysis at 65-75°C, centrifuging at 5000-7000 rpm for 5-10 min to collect the supernatant, evaporating under reduced pressure the supernatant to obtain a yeast extract paste with high base and base derivative contents (30-35% moisture content), or spray-drying to obtain a powder (< 5% moisture).

Specific sources of reagents used in Examples are listed in Table 1 below.

**Table 1. Source information of reagents and instruments used herein**

| Materials | Performance indexes | Vendors or public sources |
|---|---|---|
| Nuclease | 80000 U/g | Angel Yeast Co., Ltd. |
| Yeast extract | Reagent grade yeast extract FM888 | Angel Yeast Co., Ltd. |
| Guanine deaminase | 50000 U/g | Angel Yeast Co., Ltd. |
| Adenine deaminase | 50000 U/g | Angel Yeast Co., Ltd. |
| *Bacillus subtilis* | CCTCC 131157 | China Center for Type Culture Collection |

*Saccharomyces cerevisiae* FX-2 used in Examples of the present disclosure is deposited in the China Center for Type Culture Collection (CCTCC) on August 1, 2016, with the accession number of CCTCC NO: M2016418, at Wuhan University, Wuhan, China. This strain has been described in Patent Publication No. CN108220175A.

*Wickerhamomyces anomalus* C1.7 used in Examples of the present disclosure is deposited in the China Center for Type Culture Collection (CCTCC) on December 11, 2017, with the accession number of CCTCC NO: M2017782, at Wuhan University, Wuhan, China. This strain has been described in Patent Publication No. CN110959853A.

*Cyberlindnera fabianii* C1.8 used in Examples of the present disclosure is deposited in the China Center for Type Culture Collection (CCTCC) on December 11, 2017, with the accession number of CCTCC NO: M2017780, at Wuhan University, Wuhan, China. This strain has been described in Patent Publication No. CN110959853A.

### Example 1: Yeast extract

A yeast extract was prepared as follows:
1. Preparation of yeast culture medium: the culture medium was prepared with 100 L of purified water based on the mass ratio of raw materials to water as follows: 3% of cane molasses, 0.8% of yeast extract, 2% of ammonium sulfate, 1% of magnesium sulfate, 0.5% of potassium dihydrogen phosphate, and 0.1% of zinc sulfate. The pH was adjusted to 4.5.
2. Culture of *Saccharomyces cerevisiae* FX-2: the amount of *Saccharomyces cerevisiae* FX-2 inoculated was 1% (w/v), the culture temperature was 30°C, the aeration rate was 50 L/min, the stirring speed was 300 rpm/min, and the culture time was 12 h.
3. The culture was centrifuged at 5000 rpm for 5 min. The heavy phase was collected, to which purified water was added and stirred well and then centrifuged at 5000 rpm for 5 min. The heavy phase was collected again, and purified water was added until the dry weight of yeast cells reached 10% (w/v).
4. The above yeast milk was heated to 50°C, and the pH was adjusted to 5.0. Then, 0.1% of adenine deaminase and 1% of substrate disodium ribonucleotide were added based on the dry weight of the yeast milk. Autolysis was performed for 20 h while keeping the temperature and pH constant.
5. The autolytic solution was heated to 65°C and maintained for 30 min. The solution was centrifuged at 5000 rpm for 5 min. The supernatant was collected, concentrated by evaporation under reduced pressure at 92-95 kpa and 80°C, and spray-dried to obtain a powder finished product with a moisture content of 3.5%.

### Example 2: Yeast extract

A yeast extract was prepared as follows:
1. Preparation of yeast culture medium: the culture medium was prepared with 100 L of purified water based on the mass ratio of raw materials to water as follows: 5% of cane molasses, 0.5% of yeast extract, 4% of ammonium sulfate, 1.5% of magnesium sulfate, 0.5% of potassium dihydrogen phosphate, and 0.3% of zinc sulfate. The pH was adjusted to 5.0.
2. Culture of *Wickerhamomyces anomalus* C1.7: the amount of *Wickerhamomyces anomalus* C1.7 inoculated was 1% (w/v), the culture temperature was 30°C, the aeration rate was 50 L/min, the stirring speed was 300 rpm/min, and the culture time was 20 h.
3. The culture was centrifuged at 6000 rpm for 5 min. The heavy phase was collected, to which purified water was added and stirred well and then centrifuged at 6000 rpm for 5 min. The heavy phase was collected again, and purified water was added until the dry weight of yeast cells reached 12% (w/v).
4. The above yeast milk was heated to 40°C, and the pH was adjusted to 5.5. Then, 0.25% of nuclease, 0.1% of guanine deaminase, and 3% of substrate yeast RNA were added based on the dry weight of the yeast milk. Autolysis was performed for 15 h while keeping the temperature and pH constant.
5. The autolytic solution was heated to 65°C and maintained for 30 min. The solution was centrifuged at 5000 rpm for 5 min. The supernatant was collected, concentrated by evaporation under reduced pressure at 92-95 kpa and 80°C, and spray-dried to obtain a powder finished product with a moisture content of 3.7%.

### Example 3: Yeast extract

A yeast extract was prepared as follows:
1. Preparation of yeast culture medium: the culture medium was prepared with 100 L of purified water based on the mass ratio of raw materials to water as follows: 10% of cane molasses, 1% of yeast extract, 5% of ammonium sulfate, 2% of magnesium sulfate, 0.5% of potassium dihydrogen phosphate, and 1% of zinc sulfate. The pH was adjusted to 6.0.
2. Culture of *Cyberlindnera fabianii* C1.8: the amount of *Cyberlindnera fabianii* C1.8 inoculated was 1% (w/v), the culture temperature was 33°C, the aeration rate was 50 L/min, the stirring speed was 300 rpm/min, and the culture time was 24 h.
3. The culture was centrifuged at 6000 rpm for 5 min. The heavy phase was collected, to which purified water was added and stirred well and then centrifuged at 6000 rpm for 5 min. The heavy phase was collected again, and purified water was added until the dry weight of yeast cells reached 15% (w/v).
4. The above yeast milk was heated to 60°C, and the pH was adjusted to 6.0. Then, 0.5% of nuclease, 0.1% of adenine deaminase, 0.1% of guanine deaminase, 3% of substrate yeast RNA, and 2% of substrate disodium ribonucleotide were added based on the dry weight of the yeast milk. Autolysis was performed for 24 h while keeping the temperature and pH constant.
5. The autolytic solution was heated to 70°C and maintained for 30 min. The solution was centrifuged at 5000 rpm for 5 min. The supernatant was collected, concentrated by evaporation under reduced pressure at 92-95 kpa and 80°C, and spray-dried to obtain a powder finished product with a moisture content of 3.0%.

### Comparative Example 1: Effect of enzyme on yeast extract performance

A yeast extract was prepared as follows:
1. Preparation of yeast culture medium: the culture medium was prepared with 100 L of purified water based on the mass ratio of raw materials to water as follows: 6% of cane molasses, 0.8% of yeast extract, 3.5% of ammonium sulfate, 1% of magnesium sulfate, 0.3% of potassium dihydrogen phosphate, and 0.6% of zinc sulfate. The pH was adjusted to 5.5.
2. Yeast culture: the amount of *Saccharomyces cerevisiae* FX-2 inoculated was 1% (w/v), the culture temperature was 30°C, the aeration rate was 50 L/min, the stirring speed was 300 rpm/min, and the culture time was 20 h.
3. The culture was centrifuged at 6000 rpm for 5 min. The heavy phase was collected, to which purified water was added and stirred well and then centrifuged at 6000 rpm for 5 min. The heavy phase was collected again, and purified water was added until the dry weight of yeast cells reached 13% (w/v).
4. The above yeast milk was heated to 55°C, and the pH was adjusted to 5.5. Autolysis was performed for 20 h while keeping the temperature and pH constant.
5. The autolytic solution was heated to 65°C and maintained for 30 min. The solution was centrifuged at 5000 rpm for 5 min. The supernatant was collected, concentrated by evaporation under reduced pressure at 92-95 kpa and 80°C, and spray-dried to obtain a powder finished product with a moisture content of 4.0%.

### Comparative Example 2: Effect of enzyme usage on yeast extract performance

A yeast extract was prepared as follows:
1. Preparation of yeast culture medium: the culture medium was prepared with 100 L of purified water based on the mass ratio of raw materials to water as follows: 10% of cane molasses, 1% of yeast extract, 5% of ammonium sulfate, 2% of magnesium sulfate, 0.5% of potassium dihydrogen phosphate, and 1% of zinc sulfate. The pH was adjusted to 6.0.
2. Culture of *Cyberlindnera fabianii* C1.8: the amount of *Cyberlindnera fabianii* C1.8 inoculated was 1% (w/v), the culture temperature was 33°C, the aeration rate was 50 L/min, the stirring speed was 300 rpm/min, and the culture time was 24 h.
3. The culture was centrifuged at 6000 rpm for 5 min. The heavy phase was collected, to which purified water was added and stirred well and then centrifuged at 6000 rpm for 5 min. The heavy phase was collected again, and purified water was added until the dry weight of yeast cells reached 15% (w/v).
4. The above yeast milk was heated to 60°C, and the pH was adjusted to 6.0. Then, 0.05% of nuclease, 0.03% of adenine deaminase, 0.03% of guanine deaminase, 3% of substrate yeast RNA, and 2% of substrate disodium ribonucleotide were added based on the dry weight of the yeast milk. Autolysis was performed for 24 h while keeping the temperature and pH constant.
5. The autolytic solution was heated to 70°C and maintained for 30 min. The solution was centrifuged at 5000 rpm for 5 min. The supernatant was collected, concentrated by evaporation under reduced pressure at 92-95 kpa and 80°C, and spray-dried to obtain a powder finished product with a moisture content of 3.2%.

### Comparative Example 3: Effect of precursor usage on yeast extract performance

A yeast extract was prepared as follows:
1. Preparation of yeast culture medium: the culture medium was prepared with 100 L of purified water based on the mass ratio of raw materials to water as follows: 3% of cane molasses, 0.8% of yeast extract, 2% of ammonium sulfate, 1% of magnesium sulfate, 0.5% of potassium dihydrogen phosphate, and 0.1% of zinc sulfate. The pH was adjusted to 4.5.
2. Culture of *Saccharomyces cerevisiae* FX-2: the amount of *Saccharomyces cerevisiae* FX-2 inoculated was 1% (w/v), the culture temperature was 30°C, the aeration rate was 50 L/min, the stirring speed was 300 rpm/min, and the culture time was 12 h.
3. The culture was centrifuged at 5000 rpm for 5 min. The heavy phase was collected, to which purified water was added and stirred well and then centrifuged at 5000 rpm for 5 min. The heavy phase was collected again, and purified water was added until the dry weight of yeast cells reached 10% (w/v).
4. The above yeast milk was heated to 50°C, and the pH was adjusted to 5.0. Then, 0.5% of nuclease, 0.1% of adenine deaminase, 0.1% of guanine deaminase, 0.5% of substrate yeast RNA, and 0.2% of substrate disodium ribonucleotide were added based on the dry weight of the yeast milk. Autolysis was performed for 22 h while keeping the temperature and pH constant.
5. The autolytic solution was heated to 65°C and maintained for 30 min. The solution was centrifuged at 5000 rpm for 5 min. The supernatant was collected, concentrated by evaporation under reduced pressure at 92-95 kpa and 80°C, and spray-dried to obtain a powder finished product with a moisture content of 3.8%.

The yeast extracts prepared in Examples 1-3 and Comparative Examples 1-3 were detected for total nitrogen, amino acid nitrogen, moisture, bases and base derivatives, and the content of various bases and base derivatives. The detection methods for various physical and chemical indexes were as follows. The results are shown in Tables 2 and 3.

### (1) Determination of moisture

According to the method specified in 6.2 of the national standard GB/T 23530-2009, a certain mass of sample was dried at 103°C for 4 hours until a constant weight and then weighed to calculate the moisture content. The results are shown in Table 2.

### (2) Determination of total nitrogen

According to the Kjeldahl method specified in 6.4 of the national standard GB/T 23530-2009, a sample (equivalent to 30-440 mg of total nitrogen) was added with 20 mL of concentrated sulfuric acid for digestion in the presence of 5 g of mixed catalyst a (a mixture of potassium sulfate and copper sulfate pentahydrate at a ratio of 97:3) and 2.5 g of catalyst b (a mixture of selenium powder and potassium sulfate at a ratio of 0.1:100); distillation was performed, and the product ammonia was absorbed with boric acid; titration was then performed with 0.1 mol/L hydrochloric acid, and the data was read and calculated for the total nitrogen content. The results are shown in Table 2.

### (3) Determination of amino acid nitrogen

According to the detection method for amino acid nitrogen specified in 6.5 of the national standard GB/T 23530-2009,
5 g of sample was diluted, titrated to pH 8.2 with 0.5 mol/L sodium hydroxide solution, and then kept for 1 min. 10 mL of 36% formaldehyde solution was slowly added and reacted with non-dissociated amino groups in neutral amino acids to generate mono- and di-methylol inducers. This reaction was completely quantitative. The hydrogen ions released at this time were titrated with the above sodium hydroxide. The content of amino acid nitrogen was calculated based on the amount of the alkali solution consumed. The results are shown in Table 2.

### (4) Determination of pH

According to the detection method for pH specified in 7.2.1 of the national standard GB/T 35536-2017, a certain weight percentage concentration of aqueous solution was prepared, and the pH value was determined after sterilization using a glass electrode. The difference of two measured values of the same sample shall not exceed 0.04.

### (5) Determination of bases and base derivatives

The content of various bases and base derivatives was determined using high-performance liquid chromatography.
High-performance liquid chromatograph: equipped with UV detector and SCX column, 250 mm * 4.6 mm 5 um
Mobile phase: 0.1% phosphoric acid
Column temperature: 35°C
Detection wavelength: 267 nm

Various bases and base derivatives were prepared into a certain concentration of standard solution, injected into a high-performance liquid chromatograph, and detected to draw a standard curve. The sample to be tested was dissolved in water, passed through a 0.22 um membrane, and injected for high-performance liquid chromatography analysis. The concentration of various bases and base derivatives in the sample was calculated based on the standard curve. The results are shown in Tables 2 and 3.

**Table 2. Detection results of physical and chemical indexes**

| | Total nitrogen | Amino acid nitrogen | Bases and base derivatives | Moisture |
|---|---|---|---|---|
| Example 1 | 10.5% | 4.0% | 22000 mg/kg | 3.5% |
| Example 2 | 11.2% | 4.3% | 25000 mg/kg | 3.7% |
| Example 3 | 11.8% | 5.0% | 39000 mg/kg | 3.0% |
| Comparative Example 1 | 11.0% | 4.2% | 3000 mg/kg | 4.0% |
| Comparative Example 2 | 11.1% | 4.1% | 3300 mg/kg | 3.8% |
| Comparative Example 3 | 10.9% | 4.2% | 4500 mg/kg | 3.9% |

**Table 3. Content of bases and base derivatives**

| | Adenine (mg/kg) | Guanine (mg/kg) | Uracil (mg/kg) | Cytosine (mg/kg) | Xanthine (mg/kg) | Hypoxanthine (mg/kg) | Total (mg/kg) |
|---|---|---|---|---|---|---|---|
| Example 1 | 2000 | 4000 | 4000 | 1500 | 8000 | 2500 | 22000 |
| Example 2 | 5000 | 3000 | 4000 | 3000 | 8000 | 2000 | 25000 |
| Example 3 | 5000 | 6000 | 7000 | 5000 | 11000 | 5000 | 39000 |
| Comparative Example 1 | 500 | 500 | 500 | 500 | 1000 | 0 | 3000 |
| Comparative Example 2 | 700 | 500 | 500 | 600 | 1000 | 0 | 3300 |
| Comparative Example 3 | 800 | 700 | 800 | 700 | 1500 | 0 | 4500 |

As shown in Tables 2 and 3 above, the content of bases and base derivatives in the yeast extract prepared in Examples 1-3 was 22000-39000 mg/kg, while that in the yeast extract prepared in Comparative Examples 1-3 was 3000-4500 mg/kg, indicating that the yeast extract prepared by the method provided herein has a higher content of bases and base derivatives.

### Experimental Example 1

The yeast extracts from the above Examples 1-3, Comparative Examples 1-3, and prior art CN106282242A were used to culture *Bacillus subtilis* CCTCC 131157. The bacterial OD600 and final adenosine production were measured. The specific experimental operations were as follows: 1. Preparation of fermentation medium: the culture medium was prepared with 1 L of purified water based on the mass ratio of raw materials to water as follows: 150 g/L of glucose, 35 g/L of yeast extract powder (yeast extracts of Examples 1-3, Comparative Examples 1-3, and CN106282242A), 10 g/L of magnesium sulfate, 10 g/L of potassium dihydrogen phosphate, 0.05 g/L of manganese sulfate, and 0.01 g/L of copper sulfate, and sterilized at 121°C for 20 min. *2. Bacillus subtilis* CCTCC 131157 was inoculated into the above culture medium and cultured for 48 h. The OD600 and adenosine production were measured. The detection results are shown in Table 4 below.

**Table 4**

| | OD600 | Adenosine production |
|---|---|---|
| Example 1 | 50 | 34 g/L |
| Example 2 | 54 | 40 g/L |
| Example 3 | 62 | 45 g/L |
| Comparative Example 1 | 37 | 20 g/L |
| Comparative Example 2 | 36 | 20 g/L |
| Comparative Example 3 | 37 | 23 g/L |
| Yeast extract of CN106282242A | 42 | 26 g/L |

From Table 4 above, it can be seen that the application of the yeast extracts provided in Examples 1-3 of the present disclosure to a microbial culture medium can better promote microbial growth.

The above descriptions are only the preferred examples of the present disclosure and are not intended to limit the present disclosure, and various changes and modifications may be made by those skilled in the art. Any modification, equivalent replacement, or improvement made without departing from the spirit and principle of the present disclosure shall fall within the scope of the present disclosure.

## Claims

1. A yeast extract with high base and base derivative contents, **characterized in that** the yeast extract comprises 20000-40000 mg of bases and base derivatives per kg of yeast extract.

2. The yeast extract according to claim 1, wherein the yeast extract comprises 22000-39000 mg of bases and base derivatives per kg of yeast extract;
preferably, the bases and base derivatives comprises one or two or more selected from the group consisting of adenine, guanine, uracil, cytosine, xanthine, and hypoxanthine;
preferably, the yeast extract comprises 2000-5000 mg of adenine per kg of yeast extract;
and/or, preferably, the yeast extract comprises 3000-6000 mg of guanine per kg of yeast extract;
and/or, preferably, the yeast extract comprises 4000-7000 mg of uracil per kg of yeast extract;
and/or, preferably, the yeast extract comprises 1500-5000 mg of cytosine per kg of yeast extract;
and/or, preferably, the yeast extract comprises 8000-11000 mg of xanthine per kg of yeast extract;
and/or, preferably, the yeast extract comprises 2000-5000 mg of hypoxanthine per kg of yeast extract.

3. The yeast extract according to claims 1 or 2, wherein the yeast extract comprises a total nitrogen content of 10-12% by weight; preferably, the yeast extract comprises the total nitrogen content of 10.5-11.8% by weight;
preferably, the yeast extract comprises a nitrogen content from amino acids of 3.5-5.5% by weight; preferably, the yeast extract comprises the nitrogen content from amino acids of 4-5% by weight.

4. A method for preparing the yeast extract according to any one of claims 1-3, comprising the following steps:
heating yeast milk to 40-60°C, adjusting the pH to 5.0-6.0, adding the content of an enzyme of 0.1-1% by weight based on the dry weight of the yeast milk, adding the content of a precursor of 1-5% by weight based on the dry weight of the yeast milk, and autolyzing for 15-24 h.

5. The method according to claim 4, wherein the enzyme is one or two or more selected from the group consisting of nuclease, adenine deaminase, and guanine deaminase,
preferably, the enzyme is a combination of nuclease, adenine deaminase, and guanine deaminase; preferably, the mass ratio of the nuclease, adenine deaminase, and guanine deaminase is 2.5-5:1:1.

6. The method according to claims 4 or 5, wherein the precursor is one or two selected from the group consisting of yeast RNA or disodium ribonucleotide.

7. The method according to any one of claims 4-6, further comprising: inactivating the enzyme after autolysis, solid-liquid separation, and then concentrating the liquid to obtain the yeast extract;
preferably, the enzyme is inactivated at 65-75°C;
preferably, the concentration is performed by evaporation under reduced pressure.

8. The method according to any one of claims 4-7, wherein the yeast is one or two or more selected from the group consisting of *Saccharomyces cerevisiae, Wickerhamomyces anomalus,* and *Cyberlindnera fabianii.*

9. The method according to any one of claims 4-8, wherein the method for preparing the yeast milk comprises the following steps:
inoculating a yeast strain into a culture medium, culturing at pH 4.0-6.0 and a temperature of 30-33°C for 12-24 h, and collecting yeast cells to obtain the yeast milk.

10. The method according to claim 9, wherein in the yeast milk, the content of the yeast cells is 10-15 wt% based on dry matter content, and the rest is water.

11. The method according to claims 9 or 10, wherein the inoculation amount of yeast strain is 1-5 wt%,
preferably, the culture medium is obtained by adding 3-10 g of carbon source, 0.5-1 g of yeast extract, 2-5 g of ammonium sulfate, 1-2 g of magnesium sulfate, 0.5-1 g of potassium dihydrogen phosphate, and 0.1-0.5 g of zinc sulfate to every 100 ml of water,
preferably, the carbon source is one or two or more selected from the group consisting of cane molasses, beet molasses, or hydrolyzed sugars.

12. A yeast extract prepared by the method according to any one of claims 4-11.

13. Use of the yeast extract according to any one of claims 1-3 or claim 12 or a yeast extract prepared by the method according to any one of claims 4-11 in foods, feeds, and culture media.
